# EUROPEAN PATENT APPLICATION

(11) **EP 0 890 336 A1**
(43) Date of publication of application: **13.01.1999**
(21) Application number: 98500150.2
(22) Date of filing: 26.06.1998
(51) Int. Cl.: A47L 13/17

(54) **System for the control of the cleanliness and hygiene in hostelry, hospitals and other establishments, and the apparatus and cloths used in these establishments**

(30) Priority: 10.07.1997 ES 9701535
(71) Applicant: Guasch Pubill, Marcos, E-08005 Barcelona (ES)
(72) Inventor: Guasch Pubill, Marcos, E-08005 Barcelona (ES)
(74) Representative: Pastells Teixido, Manuel

(57) **Abstract**

Put into practice by using code marked apparel and cloths treated with a bacteriostatic and fungistatic finish so that each article of apparel and cloth is flagged to be used only for a given function and zone within the establishment, the finish treatment of the apparel and cloths being resistant to several washes. The apparel and cloths incorporate a marker or an indicator of the efficacy level of the bacteriostatic and fungistatic treatment. The code mark of the apparel and cloths consists of flagging them with different colours. Within the system, at the start of the work shift the apparel and cloths should be placed in the zones or sections of the establishment according to the corresponding colour code, and at the ends of the shift all the apparel and cloths are collected in different recipients according to their colour code.

## Description

The present invention refers to a system for the control of the cleanliness and hygiene in hostelry, hospitals and other establishments, and the apparel and cloths used in these establishments.

Currently the cleanliness and hygiene in these establishments leaves much to be desired, especially in kitchens where there are utensils, tools or surfaces that come into contact with food, either raw or cooked, and causing the proliferation of microbes and fungi, and so called cross contamination that produces infections and diseases in the persons that consume food that is not in suitable condition.

Generally when cleaning kitchens, bathrooms, bedrooms, etc. of these establishments, the same conventional cloth is used indiscriminately passing cross microbial contamination from one zone to another.

Attempts have been made to resolve this problem as far as possible using disposable cloths, however this represents considerable outlay in the cleaning budget for these establishments, and it is not an adequate solution.

This problem has been resolved by developing the system object of this invention and which uses cloths with special characteristics that allow them to be washed several times while maintaining their hygienic and cleansing function in perfect condition. This system enables rigorous control over the preparation, conservation and manipulation of foods. These characteristic can also be applied to the apparel worn by the persons carrying out these cleaning operations, such as aprons, pinafores, caps and other articles.

When this system is used in various departments of, for example, a hotel or a hospital, it will be particularly useful in all phases of the food chain: preparation, manufacture, transformation, packing, storage, transport, distribution, manipulation and sale.

The system basically consists of the use of code marked apparel and cloths (for example in different colours) and treated with a bacteriostatic and fungistatic finish so that each article of apparel or cloth is flagged to be used only for certain functions and zones within the establishment. The finish of the apparel and cloths is resistant to several washes.

This apparel and cloths also incorporate a marker (for example determined by a degradation or change of colour) or an indicator that continuously shows the efficacy level of the bacteriostatic and fungistatic finish. Another advantage of these cloths is that they also incorporate a soil release finish that simplifies the removal of dirt when they reach maximum absorption and collection of solid residues and further simplifying dirt removal during washing of the cloths.

In this system it is important that, at the start of the working session, the indicated apparel and cloths are placed according to their code in the corresponding zones or sections of the establishment, and that, at the end of the session, all the apparel and cloths be collected in different recipients according to their code.

These and other characteristics will be better understood from the following detailed description. Further clarification is provided by the drawings which represent a practical case of application that is only given as an example and does not in any way limit the scope of the present invention.

In the drawings:
Figure 1 shows a view of the cloths used in the system presented for use as a roll formed by several cloths.
Figure 2 is an elevation drawing of one of these cloths already separate from the roll, and
Figure 3 is a schematic diagram of the different phases in the manufacture of the cloth.

In this example of application the system is used in a kitchen.

A colour code is used to visually identify the correct location of the cloth 1 which is bordered by edging 2 printed with the corresponding colour.

Thus, for example, the blue colour can be used for the raw food zone, for cleaning, drying and protection of materials or surfaces that enter into contact with raw food; the red colour for the cooked food zone, for cleaning, drying and protecting utensils and surfaces that come into contact with cooked foods; and the green colour for the cleaning zone, for drying and cleaning previously disinfected surfaces and utensils.

In addition to the colour printing in each cloth, the cotton fabric used for their manufacture will have received a bacteriostatic and fungistatic finish resistant to several washes (between fifteen and twenty) and will prevent the proliferation of fungi and microbes.

Correct implementation of this system requires that each zone or section of the establishment only contains cloths with the colour assigned to that section, and no other.

When this system is applied, all the necessary cloths of the colour corresponding to each zone or section will be delivered at the start of the working session. At the end of the session all the cloths will be collected in separate recipients according to the colour. They will then be washed for later use.

Each cloth will be printed with a marker 3 that will undergo a degradation or colour change to indicate the efficacy level of the bacteriostatic and fungistatic treatment, or an indicator 3 of the number of wash cycles at different temperatures, for example 30, 60 and 90°C during which this efficacy is maintained.

According to this system re-disinfection must be carried out of all the utensils and surfaces that may have come into contact with food. If any cloth is detected as missing from the daily re-count, the reasons for this disappearance must be determined in order prevent it being found in any zone of where there is a risk of contamination. If a cloth has been used with the marker below the critical limit, this cloth must be withdrawn immediately from the circuit for cloths with a hygienic guarantee. If any cloth of a colour other than that corresponding to the section is detected it is to be immediately withdrawn from the circuit and washed at 90°C.

The system must be controlled at certain time intervals to verify that the marker is fulfilling its indicator function. Laboratory testing is also recommended to verify the efficacy of the bacteriostatic and fungistatic treatment.

In addition to their basic characteristics the cloths will also present a soil release finish to obtain maximum absorption of liquids and the collection of solid residues, and to facilitate rapid removal of the dirt during the wash. This finish does not allow dirt to be moved from one site to another.

Furthermore, these cloths will be subject to a leaching treatment so that they will be fully absorbent from the outset (without need of a previous wash), or will receive a mercerising treatment that will delay the appearance of pilling (formation of balls) and simplify ironing. They will be manufactured with 100% pure cotton from fabric with long fibres, not regenerated, so that they practically leave no fluff on the object that is being dried or cleaned, eliminating possible focuses of infection. This is further enhanced by singeing the fabric to remove any superficial fibres that could be present.

The cloths should be personalised with an insignia 4 in order to control their return if sent to a laundry and so prevent their being mixed with other cloths without the adequate hygienic treatment.

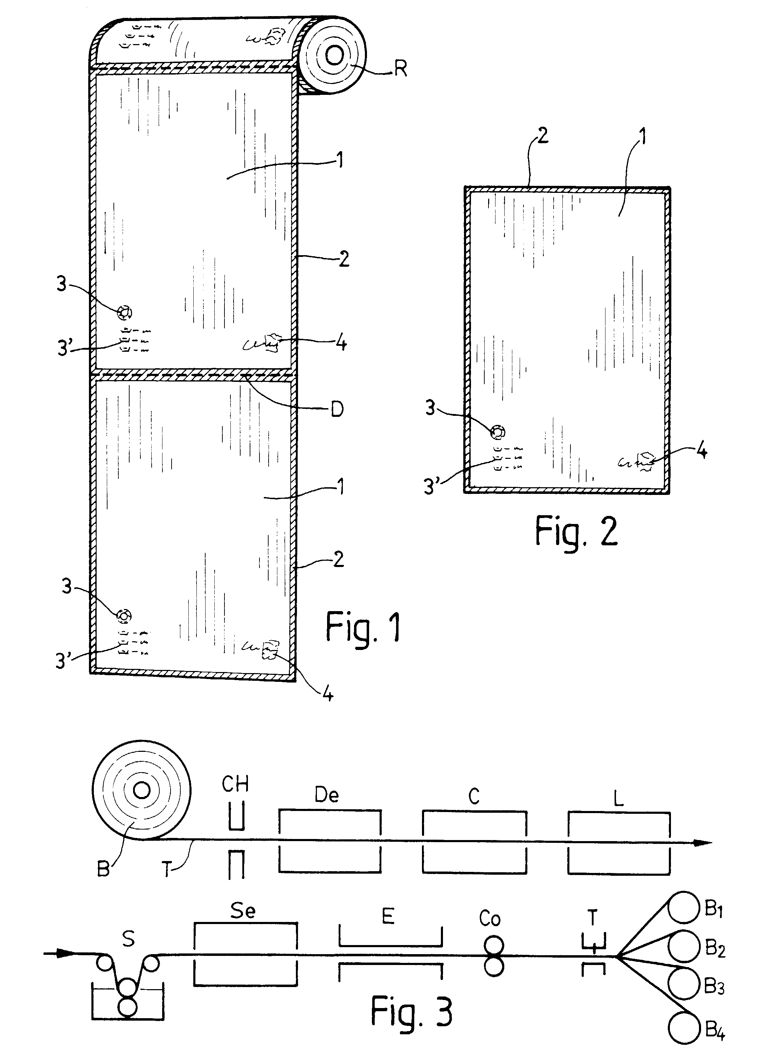

According to Figure 3 this cloth is manufactured from a fabric T supplied in the form of a bobbin B, which is as wide as a multiple of the anticipated width of the cloths. This fabric is first subject to a singeing operation CH with later de-sizing, washing and drying D. The fabric then receives a leaching or mercerising treatment C and washed, neutralised and dried L. The fabric is now ready to be given a soil release, bacteriostatic and fungistatic finish S, and dried Se. The finished fabric is then printed E with the border that will determine the colour code, the logotype that personalises it for the establishment, and the indicator of the efficacy of the bacteriostatic and fungistatic treatment. The fabric is then cut lengthways Co and then die-cut crosswise T before the series of cloths are wound onto different bobbins B1, B2, B3 and B4.

The cloths can also be presented loose packed in suitable boxes or crates, however the recommended presentation is in rolls R with a given number of attached cloths with a weakened line D that will permit easy individualisation. Logically these rolls of cloths will be fitted to a support or stand to prevent them coming into contact with unhygienic surfaces.

The apparel used in this system will have the same characteristics as those indicated for the cloths and it is understood that this system may be adapted to suit the different establishments where it is applied, according to whether they are hotels, schools, hospitals, residences, quarters, or others. A colour code should be established to distinguish the apparel and cloths for rooms, bathrooms, dining rooms or other dependencies. These codes should be different from those used for apparel and cloths in the kitchen.

The invention, in its essence, can be put into practice in other ways that only differ in detail from the above, given only as an example, and equally provide the required level of protection. This system may then be used for the control of the cleanliness and hygiene in hostelry, hospitals, and other establishments, and the apparel and cloths used in these establishments, with the most suitable means, components and accessories, being replaced by others of that are technically the same. All this is included and covered by the claims.

## Claims

1. System for control of the cleanliness and hygiene in hostelry, hospitals, and other establishments, characterised by when being put into practice the apparel and cloths used are code marked and treated with a bacteriostatic and fungistatic finish so that each article of apparel and cloth is flagged to be used only in a certain function and zone within the establishment, the finish out of the apparel and the cloths being resistant to several washes

2. System, according to claim 1, characterised by each article of apparel and cloth bearing a marker indicating the efficacy level of the bacteriostatic and fungistatic treatment.

3. System, according to claims 1 and 2, characterised by each article of apparel and cloth bearing, in substitution of the marker, an indicator of the number of washes at different temperatures that still maintain the efficacy level of the bacteriostatic and fungistatic treatment.

4. System, according to claim 1, characterised by the cloths incorporating a soil release finish that provides the cloths with a maximum absorption and collection of solid residues and simplifying dirt removal during the wash.

5. System, according to claim 1, characterissed by the cloths receiving a leach treatment that provides them with high absorbent power from the first use.

6. System, according to claims 1 and 5, characterised by instead of the leaching treatment cloths being mercerised to delay the appearance of pilling and simplify ironing.

7. System, according to claim 1, characterised by the apparel and cloths being manufactured from long fibre cotton fabrics to prevent fluff deposits during use, further enhanced by a singeing operation to burn the surface fibres.

8. System, according to claim 1, characterised by the apparel and the cloths presenting an insignia that personalises for the establishment to which they belong.

9. System, according to claim 1, characterised by the code mark of the apparel and the cloths consisting of flagging them with different colours.

10. System, according to claim 1, characterised by placing at the beginning of the work shift the apparel and cloths in the corresponding zones or sections of the establishment according to the code and at the end of the shift all the apparel and cloths being collected in different recipients according to their code.

11. System, according to claim 1, characterised by all utensils or zones being re-disinfected:
- if any article of apparel or cloth is missing from the daily recount,
- if any article of apparel or cloth has been used with the marker or the indicator below the critical limit,
- if any article of apparel or cloth is used in a zone of the establishment with a code other than that corresponding.

12. System, according to claim 2, characterised by periodical verification being made that the marker complies with its function.

13. System, according to claim 1, characterised by the cloths being supplied in rolls, one for each code, and that the cloths are separated individually.

14. Apparel and cloths to be used in the system object of claim 1, characterised by having been code marked and incorporate a bacteriostatic and fungistatic finish.

15. Apparel and cloths, according to claim 14, characterised by incorporating a marker or indicator of the level of efficacy of the bacteriostatic and fungistatic finish.

16. Apparel and cloths, according to claim 14, characterised by including an insignia that personalises them for the establishment to which they correspond.

17. Apparel and cloths, according to claim 14, characterised by the code marking consisting of a certain colour.
